# EUROPEAN PATENT SPECIFICATION

(11) **EP 0 755 277 B1**
(45) Date of publication and mention of the grant of the patent: **29.09.2004**
(21) Application number: 95903606.2
(22) Date of filing: 22.11.1994
(51) Int. Cl.: A61M 16/00, A62B 7/10, A62B 9/06, A62B 19/00, A62B 23/02, A61M 16/04

(54) **VALVED BRONCHOASPIRATION DEVICE FOR ENDOTRACHEAL TUBES**
BRONCHIALE ABSAUGVORRICHTUNG MIT EINEM VENTIL FÜR LUFTRÖHRKANÜLEN
APPAREIL DE BRONCHO-ASPIRATION A SOUPAPE DESTINE A DES SONDES ENDOTRACHEALES

(43) Date of publication of application: 29.01.1997
(73) Proprietor: MALLINCKRODT INC., St. Louis, MO 63134 (US)
(72) Inventor: GIBERTONI, Lucio, I-41037 Mirandola (IT)
(74) Representative: Modiano, Guido, Dr.-Ing.
(86) International application number: PCT/US1994/013587
(87) International publication number: WO 1995/014498

(56) References cited:
- DE-A- 2 920 366
- DE-A- 2 939 794
- US-A- 4 872 579
- US-A- 5 088 486
- US-A- 5 139 018
- US-A- 5 277 177

## Description

The present invention relates to a bronchoaspiration device applicable to an endotracheal tube in a mechanical ventilation unit.

As it is known, bronchoaspiration is a procedure required to keep the lumen of the endotracheal tube free from secretions that reduce its passage section.

This method entails a risk of bacterial contamination for the circuit and for the patient undergoing mechanical ventilation, and this risk arises mainly from the fact that a connection may form between the outside environment and the inside of the circuit during insertion of the , bronchoaspiration tube in the connector for the coupling of the endotracheal tube.

Furthermore, an additional risk is constituted by the possible repeated use of the same tube used to perform bronchoaspiration.

In the attempt to partially or fully solve these risks, currently commercially available solutions have generally led to considerable constructive complications with a consequent high cost.

Furthermore, most known devices keep inside the ventilation circuit the secretion residues that cling to the outside of the aspiration tube, thus constituting a source of additional bacterial proliferation.

DE-A-29 39 794 discloses a bronchoaspiration device comprising the features set forth in the preamble of claim 1.

US-A-4,872,579 discloses a ventilating/aspirating assembly having a valve by which aspirating vacuum pressure is selectively communicated to the interior of a catheter tube for evacuation of respiratory fluids and having an additional port for the introduction of liquids.

The aim of the invention is indeed to eliminate the above described drawbacks by providing a bronchoaspiration device that can be applied to an endotracheal tube in a mechanical ventilation unit, allows to prevent connection between the outside and the inside, and furthermore allows to remove the bronchoaspiration tube without connecting the inside to the outside.

Within the scope of the above aim, a particular object of the invention is to provide a bronchoaspiration device of the disposable type which is structurally very simple and can be applied and removed very rapidly.

Another object of the present invention is to provide a bronchoaspiration device which, by virtue of its particular constructive characteristics, is capable of giving the greatest assurances of reliability and safety in use.

Another object of the present invention is to provide a bronchoaspiration device which is easily obtainable starting from commonly commercially available elements and materials and furthermore has a very limited cost.

This aim, these objects and others which will become apparent hereinafter are achieved by a bronchoaspiration device applicable to an endotracheal tube in a mechanical ventilation unit, according to the invention, which is defined by the appended claim 1.

Further characteristics and advantages will become apparent from the description of a preferred but not exclusive embodiment of a bronchoaspiration device applicable to an endotracheal tube in a mechanical ventilation unit, illustrated only by way of non-limitative example in the accompanying drawings, wherein:

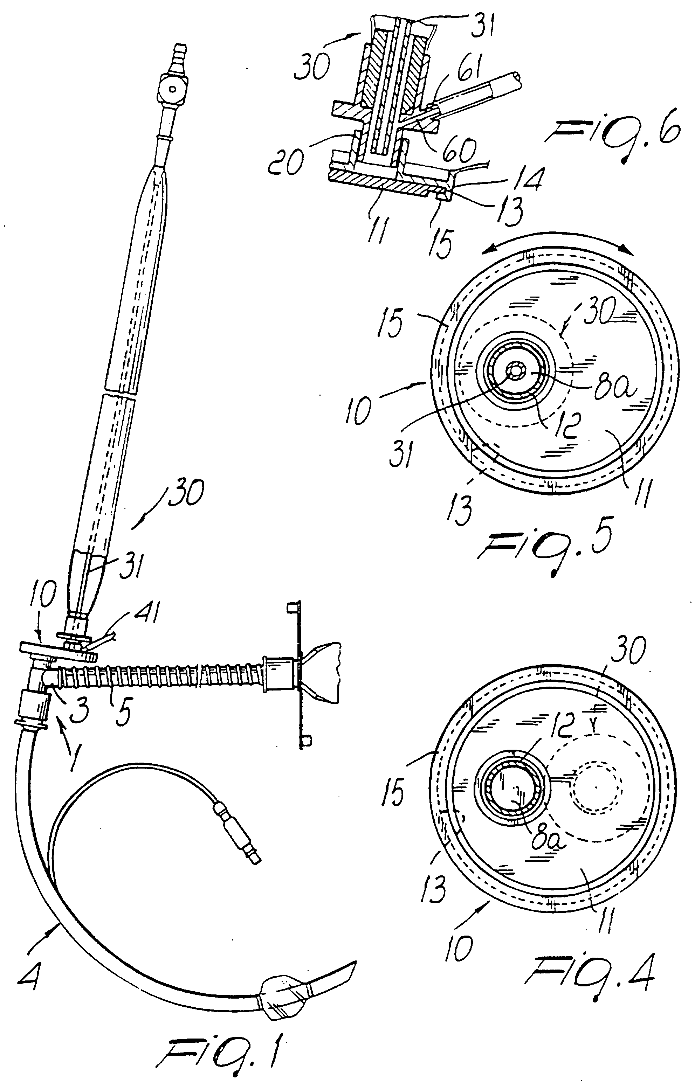

figure 1 is a schematic view of the device according to the invention;
figure 2 is a detail view of the connector with the valve element in closed position;

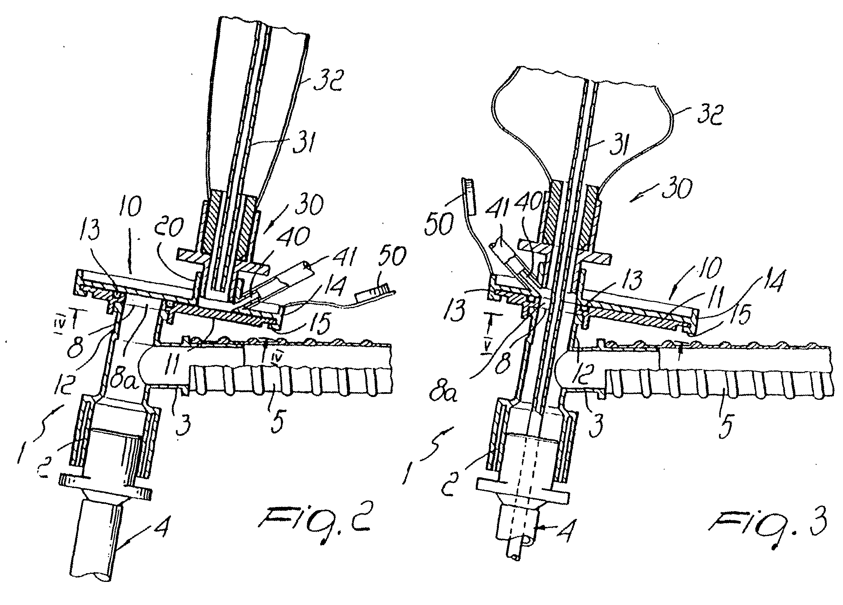

figure 3 is a partially sectional view of the connector with the valve element in aspiration position;
figure 4 is a sectional view taken along the plane IV-IV of figure 2;
figure 5 is a sectional view taken along the plane V-V of figure 3; and
figure 6 is a sectional detail view of the bronchial aspiration tube, with an auxiliary tube connected thereto for introducing liquids.

With reference to the above figures, the bronchoaspiration device applicable to an endotracheal tube in a mechanical ventilation unit, according to the invention, comprises a connector, designated by the reference numeral 1, which is commonly termed "Cobb connector" and defines an outlet port 2 and an inlet port 3 which are mutually angularly offset.

An endotracheal tube, generally designated by the reference numeral 4, is connected to the outlet port 2, whereas the air feed tube 5 is connected to the inlet port 3 and is in turn associated with a mechanical ventilation unit of a per se known type.

The particularity of the invention consists in that a lumen 8 is defined along the same axis as the outlet port 2 and is controlled by a valve element generally designated by the reference numeral 10.

More specifically, the valve element comprises a disk 11, which is connected to the cylindrical element 12 defining the lumen 8, having a sealing gasket 13, advantageously formed by an O-ring, around the lumen 8.

A rotating shutter 14 is provided above the disk 11 and has a folded lower edge 15 engaging the edge of the disk 11, so as to achieve the coupling and allow the shutter 14 to rotate with respect to the disk 11.

The shutter 14 defines an aspiration inlet 20 arranged eccentrically on said shutter, so as to match the eccentricity of the opening 8a formed at the lumen 8 provided on the connector 1.

The shutter 14, which as mentioned can rotate on the disk 11, can be arranged in a first position, or closed position, wherein the lumen 8 is closed tight by said shutter and the port 20 is also closed tight in a similar manner; a bronchoaspiration tube, generally designated by the reference numeral 30, can be applied at said port.

The tube 30 can be applied and removed without connection to the inside of the mechanical ventilation circuit.

The tube 30 is provided with an internal tube 31 accommodated inside a sterile flexible outer sheath 32 so that the aspiration tube, too, is not connected to the outside.

An auxiliary port 40 is furthermore provided at the port 20, and a small tube 41 can be connected thereto for the possible introduction of liquids normally introduced to facilitate removal of internal secretions.

A plug-like element 50 is also provided that can be arranged so as to close the port 20 when the tube 30 is not applied.

According to what is shown in figure 6, it is possible to provide an auxiliary tube, designated by the reference numeral 60, formed directly on the tang 61 of the tube 30; said tang allows coupling on the port 20. With this arrangement, in normal ventilation conditions the lumen 8 is closed tight, preventing connection between the inside of the ventilation circuit and the outside.

By turning the valve element, the inside of the sheath containing the aspiration tube is connected to the inside of the circuit, but full separation from the outside environment is maintained. In practice it is possible, also by virtue of the presence of the sheath 32, to maintain separation from the outside even while coupling the tube which, once placed at the lumen 8, can be inserted to perform bronchial aspiration.

At the end of the operation, the tube is retracted inside the sheath and the valve element is rotated into its closed position.

During this step it is possible to remove the tube in order to replace it or wash it with disinfectants, without contaminating the patient in any way.

The system as described above furthermore allows, after aspiration has ended, to remove the tube and thus create a larger maneuvering space for health-care personnel proximate to the patient's face and furthermore reduces the possibility that the patient being awake may inadvertently extubate himself.

Another advantageous aspect is the possibility to use tubes having various diameters and shapes without having to change the aspiration connector and the valve element.

From what has been described above it can be seen that the invention achieves the intended aim and objects, and in particular the fact is stressed that by virtue of extremely simple means, practically constituted by the valve element, the inside of the mechanical ventilation circuit is always assuredly kept separate from the outside, preventing any possibility of contamination, with the further advantage of allowing to remove the aspiration tube for replacement or washing.

The invention thus conceived is susceptible to numerous modifications and variations, all of which are within the scope of the inventive concept.

All the details may furthermore be replaced with other technically equivalent elements.

In practice, the materials employed, as well as the contingent shapes and dimensions, may be any according to the requirements.

## Claims

1. Bronchoaspiration device applicable to an endotracheal tube (4) in a mechanical ventilation unit, comprising a connector (1) defining an outlet port (2) for coupling an endotracheal tube (4) and defining an inlet port (3) for coupling to a mechanical ventilation unit; said connector (1) comprising a lumen (8) for a bronchoaspiration tube (30), said lumen (8) being controlled by a valve element (10) which forms an aspiration port (20) for said bronchoaspiration tube (30), said valve element (10) being arrangeable in a closed position, in which it closes said lumen (8) with respect to the outside, and in an aspiration position, wherein said aspiration port (20) is arranged at said lumen (8), said valve element (10) having a disk (11) connected to the cylindrical body (12) defining said lumen (8), a shutter (14) being rotatably provided above said disk (11) and forming said aspiration port (20); **characterized in that** the shutter (14) comprises a folded lower edge (15) engaging the edge of said disk (11) so as to achieve the coupling and allow the shutter (14) to rotate with respect to the disk (11), an auxiliary port (40) being provided on said aspiration port (20) for coupling a tube (41) for the introduction of liquids.

2. Bronchoaspiration device according to claim 1, **characterized in that** said aspiration port (20) is located eccentrically on said shutter (14) so as to match the eccentricity of said lumen (8) with respect to said disk (11).

3. Bronchoaspiration device according to one or more of the preceding claims, **characterized in that** it comprises a sealing gasket (13) arranged around said lumen (8) and acting on said shutter (14).

4. Bronchoaspiration device according to one or more of the preceding claims, **characterized in that** the aspiration tube (31) is arranged inside a flexible sheath (32) surrounding said tube (31).

5. Bronchoaspiration device according to one or more of the preceding claims, **characterized in that** it comprises a plug-like element (50) connected to said shutter (14) to removably close said aspiration port (20).

6. Bronchoaspiration device according to one or more of the preceding claims, **characterized in that** it comprises, on said bronchial aspiration tube (30), an auxiliary tube (60) for introducing liquids.

## Patentansprüche

1. Bronchiale Absaugvorrichtung, anwendbar auf einen Endotrachealtubus (4) in einer Einheit für die mechanische Beatmung, die ein Anschlussstück (1) umfasst, das eine Auslassöffnung (2) zur Verbindung mit einem Endotrachealtubus (4) bestimmt und das eine Einlassöffnung (3) zur Verbindung mit einer Einheit für die mechanische Beatmung bestimmt; wobei das Anschlussstück (1) ein Lumen (8) für einen bronchiale Absaugtubus (30) umfasst, wobei das Lumen (8) von einem Ventilelement (10) gesteuert wird, das eine Aspirationsöffnung (20) für den bronchiale Absaugtubus (30) bildet, wobei das Ventilelement (10) in einer geschlossenen Position angeordnet werden kann, in der es das Lumen (8) nach außen hin schließt, und in einer Aspirationsposition, in der die Aspirationsöffnung (20) an dem Lumen (8) angeordnet ist, wobei das Ventilelement (10) eine Scheibe (11) hat, die mit dem zylindrischen Körper (12) verbunden ist, der das Lumen (8) bestimmt, wobei eine Klappe (14) drehbar oberhalb der Scheibe (11) bereitgestellt wird und die Aspirationsöffnung (20) bildet; **dadurch gekennzeichnet, dass** die Klappe (14) eine gefaltete Unterkante (15) umfasst, die in die Kante der Scheibe (11) eingreift, um die Verbindung zu erzielen und es der Klappe (14) zu ermöglichen, sich in Bezug auf die Scheibe (11) zu drehen, wobei eine Zusatzöffnung (40) an der Aspirationsöffnung (20) bereitgestellt wird, um einen Tubus (41) zum Einführen von Flüssigkeiten zu verbinden.

2. Bronchiale Absaugvorrichtung nach Anspruch 1, **dadurch gekennzeichnet, dass** die Aspirationsöffnung (20) exzentrisch an der Klappe (14) angebracht ist, um der Exzentrizität des Lumens (8) in Bezug auf die Scheibe (11) zu entsprechen.

3. Bronchiale Absaugvorrichtung nach einem oder mehreren der obigen Ansprüche, **dadurch gekennzeichnet, dass** sie eine abdichtende Dichtung (13) umfasst, die um das Lumen (8) herum angeordnet ist und auf die Klappe (14) einwirkt.

4. Bronchiale Absaugvorrichtung nach einem oder mehreren der obigen Ansprüche, **dadurch gekennzeichnet, dass** der Absaugtubus (31) in einer flexiblen Ummantelung (32) angeordnet ist, die den Tubus (31) umgibt.

5. Bronchiale Absaugvorrichtung nach einem oder mehreren der obigen Ansprüche, **dadurch gekennzeichnet, dass** sie ein stöpselähnliches Element (50) umfasst, das mit der Klappe (14) verbunden ist, um entnehmbar die Aspirationsöffnung (20) zu verschließen.

6. Bronchiale Absaugvorrichtung nach einem oder mehreren der obigen Ansprüche, **dadurch gekennzeichnet, dass** sie an dem bronchialen Absaugtubus (30) einen Zusatztubus (60) zum Einführen von Flüssigkeiten umfasst.

## Revendications

1. Appareil de broncho-aspiration destiné à une sonde endotrachéale (4) dans une unité de ventilation mécanique, comprenant un raccord (1) définissant un port de sortie (2) pour coupler une sonde endotrachéale (4) et définissant un port d'entrée (3) pour couplage à une unité de ventilation mécanique ; ledit raccord (1) comprenant une lumière (8) pour une sonde de broncho-aspiration (30), ladite lumière (8) étant contrôlée par un élément de valve (10) qui forme un port d'aspiration (20) pour ladite sonde de broncho-aspiration (30), ledit élément de valve (10) pouvant être arrangé en une position fermée, dans laquelle il ferme la dite lumière (8) par rapport à l'extérieur, et en une position d'aspiration, dans laquelle ledit port d'aspiration (20) est arrangé au niveau de ladite lumière (8), ledit élément de valve (10) ayant un disque (11) raccordé au corps cylindrique (12) définissant ladite lumière (8), un obturateur (14) étant fourni pouvant tourner au-dessus du dit disque (11) et formant ledit port d'aspiration (20) ; **caractérisé en ce que** l'obturateur (14) comprend un bord inférieur plié (15) engageant le bord du dit disque (11) de manière à obtenir le couplage et à permettre à l'obturateur (14) de tourner par rapport au disque (11), un port auxiliaire (40) étant fourni sur ledit port d'aspiration (20) pour le couplage d'une sonde (41) pour l'introduction de liquides.

2. Appareil de broncho-aspiration selon la revendication 1, **caractérisé en ce que** ledit port d'aspiration (20) est situé de manière excentrique sur ledit obturateur (14) de manière à correspondre à l'excentricité de ladite lumière (8) par rapport au dit disque (11).

3. Appareil de broncho-aspiration selon une ou plusieurs des revendications précédentes, **caractérisé en ce qu'**il comprend un joint étanche (13) arrangé autour de ladite lumière (8) et agissant sur ledit obturateur (14).

4. Appareil de broncho-aspiration selon une ou plusieurs des revendications précédentes, **caractérisé en ce que** la sonde d'aspiration (31) est arrangée à l'intérieur d'une gaine flexible (32) entourant ladite sonde (31).

5. Appareil de broncho-aspiration selon une ou plusieurs des revendications précédentes, **caractérisé en ce qu'**il comprend un élément de type prise (50) raccordé au dit obturateur (14) pour fermer de manière amovible ledit port d'aspiration (20).

6. Appareil de broncho-aspiration selon une ou plusieurs des revendications précédentes, **caractérisé en ce qu'**il comprend, sur ladite sonde d'aspiration bronchique (30), une sonde auxiliaire (60) pour introduire des liquides.
